# EUROPEAN PATENT APPLICATION

(11) **EP 4 843 614 A1**
(43) Date of publication of application: **25.11.2026**
(21) Application number: 26180771.3
(22) Date of filing: 22.05.2026
(51) Int. Cl.: A61N 1/39

(54) **RUGGEDIZED DEFIBRILLATOR CASE**

(30) Priority: 23.05.2025 US 202563811283 P; 21.05.2026 US 202619684740
(71) Applicant: Stryker Corporation, Portage, MI 49002-9711 (US)
(72) Inventor: WEGMANN, Brittany Kendra, Portage, 49002-9711 (US); AHMED, Aamish, Portage, 49002-9711 (US); BRUMMETT, Jeremy Edward, Portage, 49002-9711 (US); CHESTER, Steven Mark, Portage, 49002-9711 (US); GERST, Madeleine Elizabeth, Portage, 49002-9711 (US); GUPTA, Ankit, Portage, 49002-9711 (US); ICINGIR, Deniz, Portage, 49002-9711 (US); MCDONALD, Brooke Allyn, Portage, 49002-9711 (US); SAUTTER, Kirk Alan, Portage, 49002-9711 (US); SHIELDS, Alison Adams Zdan, Portage, 49002-9711 (US); SOHN, Dennis Changmin, Portage, 49002-9711 (US); TAYLOR, Tyson G., Portage, 49002-9711 (US); TROYER, Jeffrey Lee, Portage, 49002-9711 (US); WONG, Jeremy G., Portage, 49002-9711 (US)
(74) Representative: Kilburn & Strode LLP

(57) **Abstract**

A ruggedized defibrillator case including a first rigid platform for storing an automated external defibrillator (AED) and accessory devices such as electrodes. The exterior of the first rigid platform may include a light pipe, a rotating handle, a detachable shoulder strap, a vent, and a latch. In some aspects, the AED may be attached to the inside of the case using a mounting bracket. The accessory devices may be stored with the AED or in a separate container included within the first rigid platform. The first rigid platform may be waterproof, watertight, water resistant, and/or buoyant.

## Description

### CROSS-REFERENCE TO RELATED APPLICATION

This application claims priority to and benefit of U.S. Provisional Patent Application No. 63/811, 283 filed May 23, 2025, the entire contents of which are incorporated herein by reference.

### BACKGROUND

During an adverse cardiac event, such as cardiac arrest, time is of the essence. Survival rates are substantial if treatment starts within the first few minutes, but delays in care can lead to brain damage and death. Conventional emergency medical service response cannot be reliably achieved in the first five minutes after collapse of a subject. Efforts to address this include the presence of automated external defibrillators (AEDs) in publicly accessible places.

AEDs may be used to provide rapid defibrillation to individuals experiencing sudden cardiac arrest. Generally, AEDs include electrode pads, a battery, a capacitor, and a processor that analyzes the heart rhythm. The electrode pads are generally small adhesive pads with electrodes that can be attached to the chest of the patient. They are connected to the AED and information obtained by the electrodes and transmitted to the AED is used to determine whether a shock is indicated. If a shock is indicated, the user is prompted to initiate the transmission of an electrical impulse (a shock) through the electrodes to the individual being treated.

While AEDs generally include instructions, the design and accessibility of AEDs and the various components contribute to response time. During emergency situations, the ability to intuitively use a device and rapidly access the various components decreases response time, impacting survival rates and patient outcomes.

### SUMMARY

According to an aspect is provided a system for storing an automated external defibrillator (AED) and accessory devices. The system comprises a first rigid platform. The first rigid platform extends longitudinally between a first opposite end and a second opposite end. The first rigid platform extends laterally between a first opposite side and a second opposite side. The first rigid platform comprises a first longitudinal half comprising a first recessed portion. The first rigid platform comprises a second longitudinal half comprising a second recessed portion. The rigid platform is configured to be folded along a longitudinal midline. A vent extends between an interior of the rigid platform and an external environment. The first recessed portion of the first rigid platform is configured to have the accessory devices disposed therein. The accessory devices can be configured to be disposed in the first recessed portion of the first rigid platform. The second recessed portion of the first rigid platform is configured to have the AED secured therein, such as via a bracket mount. The AED can be configured to be secured in the second recessed portion of the first rigid platform, such as via a bracket mount.

Optionally, the system further comprises a light pipe. The light pipe can be disposed on an outer surface of the rigid platform. The light pipe can be configured to show a status of the AED.

Optionally, the system further comprising a rotating handle. The rotating handle can be attached to the rigid platform, such as along the longitudinal midline. Optionally the rotating handle is attached to a hinge connecting the first longitudinal half and the second longitudinal half of the first rigid platform.

Optionally, the system further comprises a detachable shoulder strap. The detachable shoulder strap can be attached to the rigid platform, such as along the longitudinal midline. Optionally, the detachable shoulder strap is attached via a removable pin.

Optionally, the first rigid platform has an opening profile of about 145° to about 190°. The opening profile of the first rigid platform can e.g. be about 169°.

Optionally, the system further comprising a gasket positioned between the first opposite side and the second opposite side when the first rigid platform is in a closed configuration.

Optionally, the first rigid platform further comprises a foot. The foot can protrude from a bottom surface of the first rigid platform. The foot can protrude parallel to a hinge attached along the longitudinal extension of the first rigid platform.

Optionally, the vent is within the foot. Optionally, the vent is a protective vent or auto pressure release valve.

Optionally, the first longitudinal half and the second longitudinal half of the rigid platform are configured to be secured together using a spring-loaded latch.

Optionally, the first rigid platform comprises acrylonitrile butadiene styrene, thermoplastic polyurethane, polypropylene, polystyrene, nylon, polycarbonate, polyethylene terephthalate, or methacrylate.

Optionally, the system further comprises a visually masked storage component disposed in the first recessed portion of the rigid platform and comprising a door. The system can be configured to store the accessory devices between the door and an interior surface of the first recessed portion of the first rigid platform. The accessory devices can be configured to be stored between the door and an interior surface of the first recessed portion of the first rigid platform.

Optionally, the system further comprising accessory devices stored in an accessory device package in the first recessed portion. The accessory device package can comprise a second rigid platform in a folded configuration. The second rigid platform can extend longitudinally between a first opposite end and a second opposite end. The second rigid platform can extend laterally between a first opposite side and a second opposite side. The second rigid platform can be configured to be folded along a midline of the lateral extension. The accessory device package can comprise a first recessed portion disposed in a first lateral half of the second rigid platform. The accessory device package can comprise a second recessed portion disposed in a second lateral half of the second rigid platform. The first recessed portion of the second rigid platform can comprise a space for a first accessory. The second recessed portion of the second rigid platform can comprise a space for a second accessory device. The accessory device package can comprise a first foil seal covering the first recessed portion and the first accessory device in the second rigid platform. The accessory device package can comprise a second foil seal covering the second recessed portion and the second accessory device in the second rigid platform. The accessory device package can comprise a spring-loaded hinge at the midline of the lateral extension of the second rigid platform. The accessory device package can be enclosed in a sealable pouch.

According to an aspect is provided a method of decreasing response time for defibrillation. The method comprises enclosing, by a spring-loaded first rigid platform in a folded configuration under tension, a defibrillator. The spring-loaded first rigid platform can extend longitudinally between a first opposite end and a second opposite end. The spring-loaded first rigid platform can extend laterally between a first opposite side and a second opposite side. The first rigid platform can be configured to be folded along a longitudinal midline. The method can comprise outputting, such as by a light pipe, e.g. on a first outer surface of the spring-loaded first rigid platform, a status of the defibrillator. The method can comprise releasing the tension of the spring-loaded first rigid platform in a folded configuration, thereby providing access to a defibrillator system located within the folded configuration. The method can comprise separating, such as by the spring-loaded first rigid platform, the defibrillator from an interior of the spring-loaded first rigid platform.

Optionally, releasing the tension of the spring-loaded first rigid platform comprises unfolding the spring-loaded first rigid platform.

Optionally, the spring-loaded first rigid platform has a first recessed portion disposed in a first longitudinal half of the spring-loaded first rigid platform. Optionally, the spring-loaded first rigid platform has a second recessed portion disposed in a second longitudinal half of the spring-loaded first rigid platform. The second recessed portion can comprise a space for the defibrillator. Optionally the spring-loaded first rigid platform comprises at least one of a quick-detach handle, a detachable shoulder strap, a foot, a non-skid base, and a vent.

It will be appreciated that any of the aspects, features and options described herein can be combined. It will particularly be appreciated that any of the aspects, features and options described in view of the system apply equally to the method, and vice versa.

### BRIEF DESCRIPTION OF THE DRAWINGS

FIG. 1 illustrates an exemplary ruggedized defibrillator case for an automated external defibrillator (AED) and AED accessory devices.
FIG. 2 illustrates a top-down view of the exemplary ruggedized defibrillator case of FIG. 1.
FIG. 3 illustrates an exemplary quick-release fastening for the ruggedized defibrillator case of FIG. 1
FIG. 4 illustrates an oblique side view of the exemplary ruggedized defibrillator case of FIG. 1.
FIG. 5 illustrates an oblique end view of the exemplary ruggedized defibrillator case of FIG. 1.
FIG. 6 illustrates an exemplary attachment mechanism for an AED within a ruggedized defibrillator case.
FIG. 7 illustrates an oblique view of an AED attached to a mounting bracket.
FIG. 8 illustrates the insertion of an AED into an exemplary ruggedized defibrillator case.
FIG. 9 illustrates a top-down open view of the exemplary ruggedized defibrillator case of FIG. 8 with the AED installed.
FIG. 10 illustrates a partially open oblique view of an exemplary ruggedized defibrillator case including an AED.
FIG. 11 illustrates a side view of a partially open exemplary ruggedized defibrillator case including an AED.
FIG. 12 illustrates a side view of an open exemplary ruggedized defibrillator case including an AED.
FIG. 13 illustrates a partially open view of a ruggedized defibrillator case for an AED including the AED and an accessory device.
FIG. 14 illustrates an example of the removal of an accessory device package from a ruggedized defibrillator case for an AED.
FIG. 15 illustrates an example of an automated external defibrillator (AED) system including a ruggedized defibrillator case, an AED, and an accessory device package containing a set of electrodes.
FIG. 16 illustrates an exemplary medical accessory device receptacle.
FIG. 17 illustrates an example system for determining the viability of a medical accessory device.
FIG. 18 illustrates an example of a medical accessory device connected to an external defibrillator configured to perform various functions described herein.

### DETAILED DESCRIPTION

Various implementations described herein relate to methods and systems for storing medical devices such as automated external defibrillators (AEDs) and accessory devices that are used with the medical devices. In some aspects, the accessory devices may be electrode pads including electrodes. According to various implementations, a medical device is stored with one or more accessory devices in a ruggedized case.

As used herein, the terms "standalone medical device," "medical device," and their equivalents, can refer to an apparatus that utilizes an accessory device to monitor or to administer a treatment to a patient. In many cases, a standalone medical device is self-powered (e.g., via a battery or a means to connect to mains current), includes input devices (e.g., buttons, touchscreen, etc.) configured to receive user input signals, includes output devices (e.g., speakers, displays, etc.) configured to output signals to users, and includes processing capabilities (e.g., at least one processor) configured to analyze data, report patient conditions, recommend therapy administration, generate a therapy to be administered, or any combination thereof. Examples of standalone medical devices include monitor-defibrillators, AEDs, ventilators, and patient monitors. In various examples, standalone medical devices are reusable.

As used herein, the terms "treatment," "therapy," and their equivalents, may refer to a substance, force, or signal that can be administered to the subject for the purpose of resolving a pathology and/or reducing a symptom. Examples of treatments include the administration of one or more electrical shocks (e.g., defibrillation shocks, pacing pulses, etc.) to resolve a cardiac arrhythmia. Other types of treatments include the administration of assisted ventilation, chest compressions, medications, and the like.

Various implementations of the present disclosure will be described in detail with reference to the drawings, wherein like reference numerals present like parts and assemblies throughout the several views. Additionally, any examples set forth in this specification are not intended to be limiting and merely set forth some of the many possible implementations.

Implementations of a ruggedized defibrillator case as described herein provide a means for transporting an AED and AED accessory devices. In some aspects, the ruggedized defibrillator case includes a first rigid platform with one or more attachments and exterior features including a handle, shoulder strap, light pipe, vent, and an exterior latch or other fastening mechanism. The interior of the ruggedized defibrillator case may include a number of features for the storage of the AED and accessory devices. For example, an attachment mechanism for holding the AED and/or accessory devices in place in their respective locations within the ruggedized defibrillator case. In some aspects, the attachment mechanism may be a bracket mount that allows for the insertion and removal of the AED and/or accessory devices without the use of tools. For example, the AED and/or accessory devices may be attached to the ruggedized defibrillator case via a snap fit, compression fit, or an interference fit. In some aspects, the fit may be spring-loaded. In other aspects, the interior of the ruggedized defibrillator case may include doors or other compartment closure devices that close off one or more portions of the interior of the ruggedized defibrillator case. Such compartments may be used, for example, to hold the AED and/or accessory devices in place. The ruggedized defibrillator case may additionally include one or more sensors on the interior and/or exterior of the device. Such sensors may provide information regarding the case and, by inference, the AED and accessory devices' exposure to environmental conditions as well as the state of the AED and accessory devices.

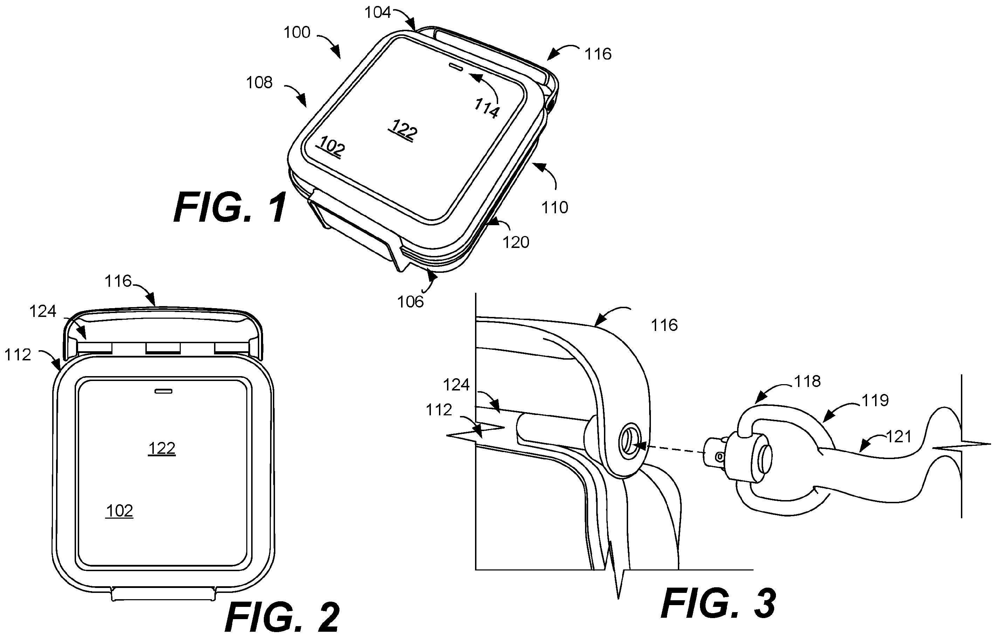

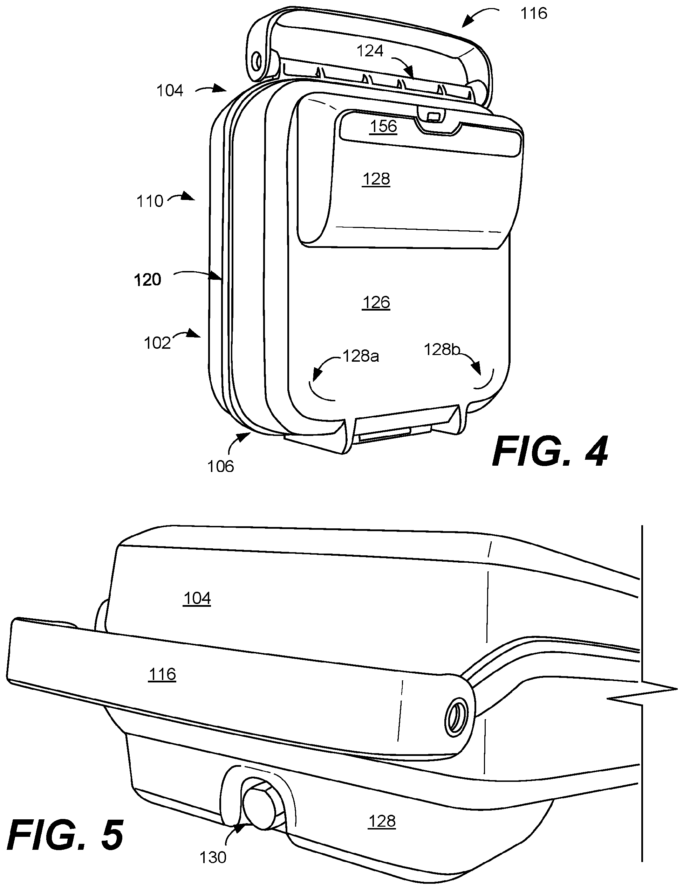

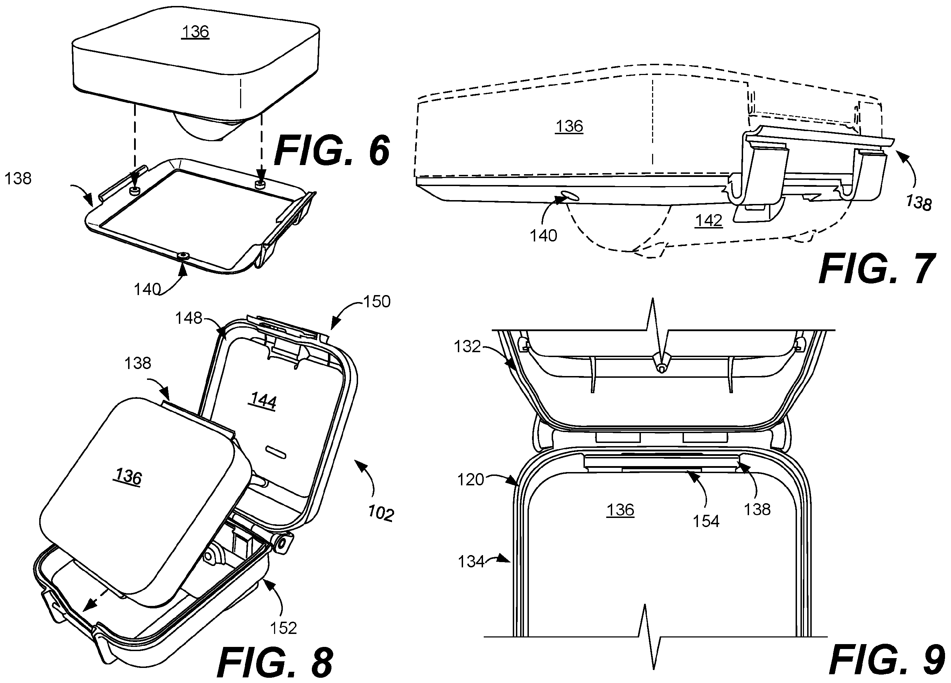

FIG. 1 Illustrates an exemplary ruggedized defibrillator case 100 including a first rigid platform 102 attached to a handle 116. The first rigid platform 102 extends longitudinally between a first opposite end 104 and a second opposite end 106 and laterally between a first opposite side 108 and a second opposite side 110. The first rigid platform 102 is configured to be folded along a midline 112 of the longitudinal extension (also referred to as a "longitudinal midline") as shown in FIG. 2 and FIG. 3 and when in an open configuration, the first rigid platform 102 is configured to allow access to an AED as well as AED accessory devices such as electrode pads. In some aspects, there may be a gasket such as a gasket 120 along the circumference of the first rigid platform 102, between the lower edge of the top 122 and the upper edge of the bottom 126 (as shown in FIG. 4 and FIG. 9) of the rigid platform.

The ruggedized defibrillator case may be made of one or more materials. For example, the handle 116 and the first rigid platform 102 may be made of the same or different materials such as polypropylene, polystyrene, nylon, polycarbonate acrylonitrile butadiene styrene, polyethylene terephthalate, thermoplastic polyurethane, or methacrylate. In some aspects, the materials may include a fire retardant. In some aspects, the ruggedized defibrillator case is water resistant. In some aspects, the ruggedized defibrillator case may be watertight. In other aspects, the ruggedized defibrillator case may be waterproof. For example, the ruggedized defibrillator case may be waterproof to a certain depth, such as less than 10 m. In some aspects, the ruggedized defibrillator case 100 may be buoyant. In some aspects, the first rigid platform 102 may include a pressure release such as an auto pressure release valve or vent as shown for example in FIG. 5. The pressure release or vent may allow for air to escape when the device is submerged or when other pressure changes are encountered such as in an airplane.

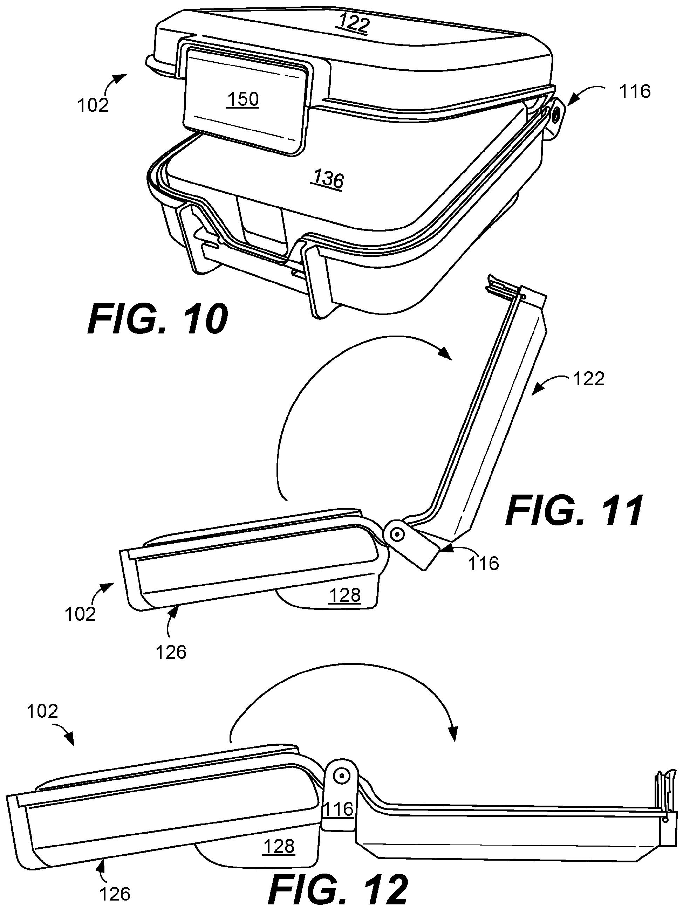

The handle 116 may be attached at or near a hinge 124 of the first rigid platform 102 as shown in FIG. 2. In some aspects, the hinge may be a spring-loaded hinge including, for example, a shape-memory alloy, a shape-memory polymer, a compression spring, a double torsion spring or a technical spring. In some aspects, the handle may be made of a flexible material such as a material with a shore hardness of 70 shore A to 100 shore A, particularly 90 shore A. In some aspects, the handle may rotate. The handle 116 may be attached such that it does not interfere with the opening and closing of the device. For example, the positioning of the handle may be such that the first rigid platform 102 can open more than 180° such as to 190° or 200° or any fraction thereof as shown in more detail with reference to FIGs. 11 and 12. In some aspects, the handle may rotate about 80°to 210°, for example about 160° to about 210° or any fraction thereof.

In some aspects, the first rigid platform 102 may have a shoulder strap 121 attached to it. The shoulder strap 121 may be attached via loop 119 of detachment mechanism 118 as shown in FIG. 3. The detachment mechanism 118 may be attached to the handle 116 via any means generally used including through the use of a pin as shown. In some aspects, the pin or the socket for the pin may include ball bearings and a spring.

The exterior of the first rigid platform 102 may also include a light pipe such as light pipe 114 or other display device. In some aspects, the light pipe 114 may provide information regarding the state or status of the first rigid platform 102 as well as devices contained within the rigid platform such as an AED or AED accessory devices. For example, the light pipe 114 may provide information regarding the expiration of the first rigid platform 102 or one or more of the devices within the first rigid platform 102. In some aspects, it may provide information regarding the readiness of the AED, any damage the AED may have sustained, the charge of the AED, and any environmental exposure that may impact the functioning of the AED and accessory devices. For example, the light pipe may convey information such as interior or exterior water damage and the like.

As shown in FIG. 4, in some aspects, the underside or bottom 126 of the first rigid platform 102 may include a foot or feet which raise the first rigid platform from any surface upon which it has been placed. While the foot 128 is shown as a single piece, in some aspects, the foot or feet may be a plurality of raised portions or edges as shown, for example, by 128a and 128b. In some aspects, the foot may include a non-skid surface such as non-skid surface 156, covering all or a portion of the foot. In some aspects, the foot may raise the bottom of the ruggedized defibrillator evenly, that is, each foot may raise a portion of the rigid platform 102 the same distance. In other aspects, as shown, the presence of a foot such as foot 128 may position the first rigid platform 102 at an angle such that the first opposite end 104 is raised. In some aspects, the foot may be hollow, providing for storage of a device or portion of a device within the first rigid platform 102. In other aspects, the foot may be of a solid construction.

In some aspects, the ruggedized defibrillator case may include a vent 130 as shown in FIG. 5. Such a vent may be an auto pressure-release valve, pressure-release valve, or other regulating mechanism. The vent 130 may allow gases and or liquids to pass between the interior and the exterior of the first rigid platform 102. For example, when the ruggedized defibrillator case is taken into pressurized areas such as within an airplane, the presence of the vent may allow the pressure within the ruggedized defibrillator case to equalize. In some aspects, the vent may allow bi-directional passage of air and liquids. In other aspects, it is unidirectional. In some aspects, the valve or vent may be an IP68 valve. While the vent 130 may be located in various locations on the ruggedized defibrillator case, in some aspects it is located in the foot 128 as shown in FIG. 5.

An AED may be fastened within the first rigid platform 102 using any mechanism generally used. In some aspects, the AED may be fastened using a mounting bracket and a mounting clip as shown in more detail with reference to FIGs. 6-9.

As shown in FIG. 6, an AED 136 may be attached to a mounting bracket 138. While the mounting bracket is depicted as being fastened to the AED using a plurality of screws 140 inserted upwards from the underside of the mounting bracket, any fastener may be used. As shown in FIG. 7, the mounting bracket 138 (solid lines) surrounds the perimeter of the AED 136 (shown in dotted lines) with a capacitor 142 of the AED 136 extending through the middle of the mounting bracket 138.

The AED 136 may be installed into or removed from the first rigid platform 102 as shown in FIG. 8. As can be seen in FIG. 8, the latch 150 has been released showing a first recessed portion 148 disposed in a first longitudinal half and a second recessed portion 152 disposed in a second longitudinal half of the first rigid platform 102. In some aspects, the latch is spring-loaded. In other aspects, the latch may be a cam latch, compression latch, draw latch, sliding latch, twist locks and the like. While the visually masked storage compartment 144 including a door for the accessory device package is shown in the first recessed half and the AED 136 is being inserted into the second recessed portion 152, other configurations are also contemplated, for example the AED 136 may be in the first recessed half. In some aspects, the top 122 and the bottom 126 of the first rigid platform 102 may each have an outer lip 132 and 134 respectively. One of the outer lip 132 or the outer lip 134 includes a gasket 120 providing a seal between the top 122 and the bottom 126 of the first rigid platform 102. In some aspects, the gasket 120 may assist in maintaining water tightness of the first rigid platform.

As shown in FIG. 9, the mounting bracket 138 is attached to the first rigid platform 102 via a fastening mechanism such as a bracket clip 154 within the first rigid platform 102. The fastening mechanism may be any mechanism that allows the AED 136 to be removed from the first rigid platform 102 without the use of tools. For example, the AED 136 and mounting bracket 138 may be fastened within the first rigid platform 102 using a snap fit, interference fit, or the like.

FIGs. 10-12 depict side views of the first rigid platform 102 showing the opening profile. As shown in FIG. 10, when the first rigid platform 102, is opened, the AED 136 is visible. As shown in FIGs. 11 and 12, the exterior rotating handle 116 is situated such that it does not interfere with opening the first rigid platform 102 such that the AED 136 and accessory devices can be accessed when the first rigid platform 102 is partially open as shown in FIG. 11 and/or when the first rigid platform 102 is fully open as shown in FIG. 12. In some aspects, the first rigid platform has an opening profile of about 145° to about 190°. For example, FIG. 12 depicts the first rigid platform 102 as being open more than 180°. In other aspects, the first rigid platform 102 may have a smaller opening profile such as 169°.

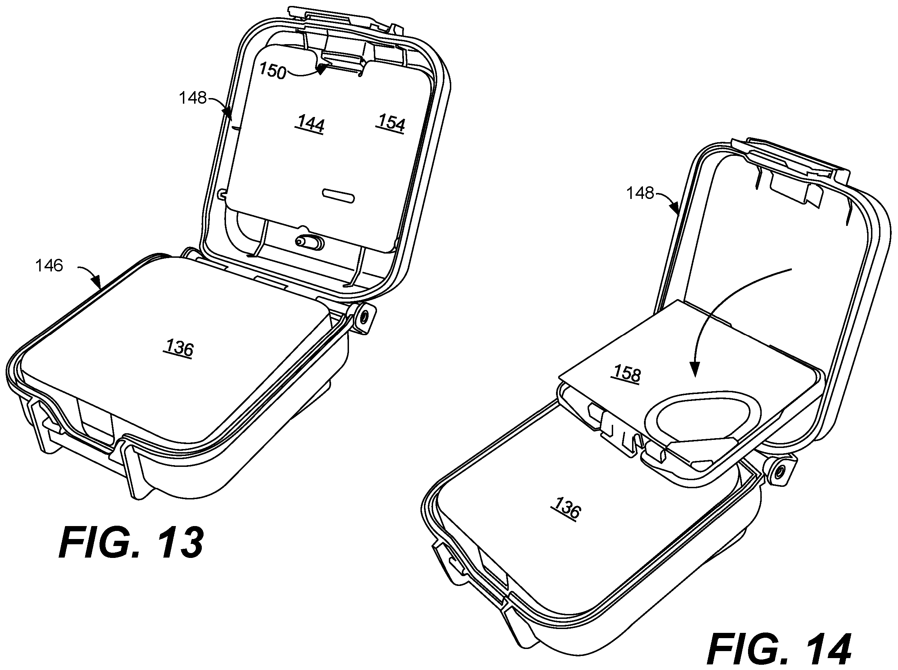

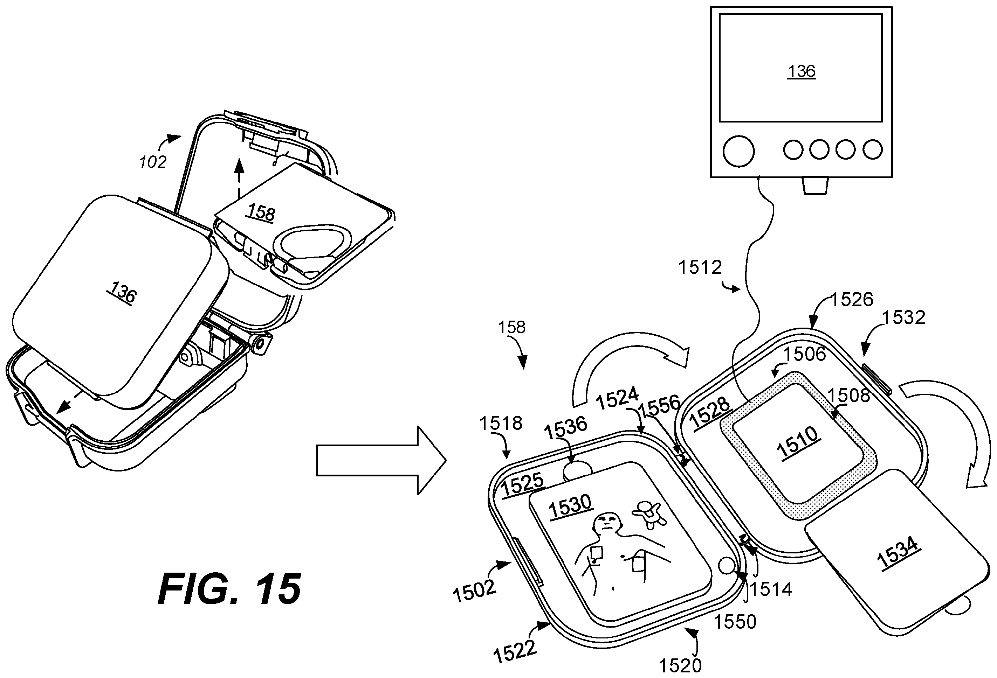

As shown in FIGs. 13 and 14, when the first rigid platform 102 is open, the AED 136 and the accessory devices are accessible. The AED 136 may be stored in a second recessed portion 152 and the accessory device package 158 may be fastened into the first recessed portion 148 of the first rigid platform 102 using any means generally used. In some aspects, the accessory device package 158 may be hidden behind a door of a visually masked storage compartment such as visually masked storage compartment 144. The door may be held closed, for example, using a latch such as latch 150. As shown in FIG. 14, the door of the visually masked storage compartment 144 and the accessory device may open in the direction of the arrow, allowing access to the accessory device package 158 behind the door of the visually masked storage compartment 144. Both the AED 136 and the accessory device package 158 may be removed from the first rigid platform 102 to be used to treat a patient as shown in further detail in the system of FIG. 15.

As shown in FIG. 15, the first rigid platform 102 contains a removable AED 136 and an accessory device within a removable package 158. In some aspects, the package 158 may substantially protect the accessory devices from dust, water, and other contaminants. In various cases, the packaging 158 may maintain an internal humidity that prevents evaporation of water from electrode pads such as those shown in FIG. 15. While the accessory devices and the accessory device packaging 158 may be presented in a variety of formats including an open tray format in which accessory devices such as electrodes are placed side-by side in a single layer, in some aspects, the accessory device package 158 may include a second rigid platform 1502.

The second rigid platform 1502 is configured to contain at least a first electrode pad (beneath protective covering 1530) and a second electrode pad 1506. Accessory devices, including devices removably configured to an AED and configured to administer a treatment and/or detect a physiological parameter, for example electrode pads, may include sticky pads that are applied to the skin on the patient's chest. Such accessory devices may relay information regarding heart rhythm (e.g., by detecting an electrical signal indicative of an electrocardiogram (ECG) of the patient) to the AED and, when appropriate, may be used to apply a shock to the patient in an effort to restore the normal rhythm of the heart. The electrode pads may be made of one or more materials. In some aspects, the electrode pads may have an adhesive layer 1508 that surrounds the electrode 1510. In some aspects, the electrodes such as a first electrode pad and a second electrode pad 1506 may be connected to wires 1512 which may extend from one or more connectors configured to plug into an AED 136. The electrodes may be disposable or reusable. For example, they may include an electrically conductive hydrogel on the surface of the electrode or in a gel pack to be applied to the surface of a dry electrode. The hydrogel, for instance, may enhance the electrical coupling between the electrode 1510 and the skin of the patient.

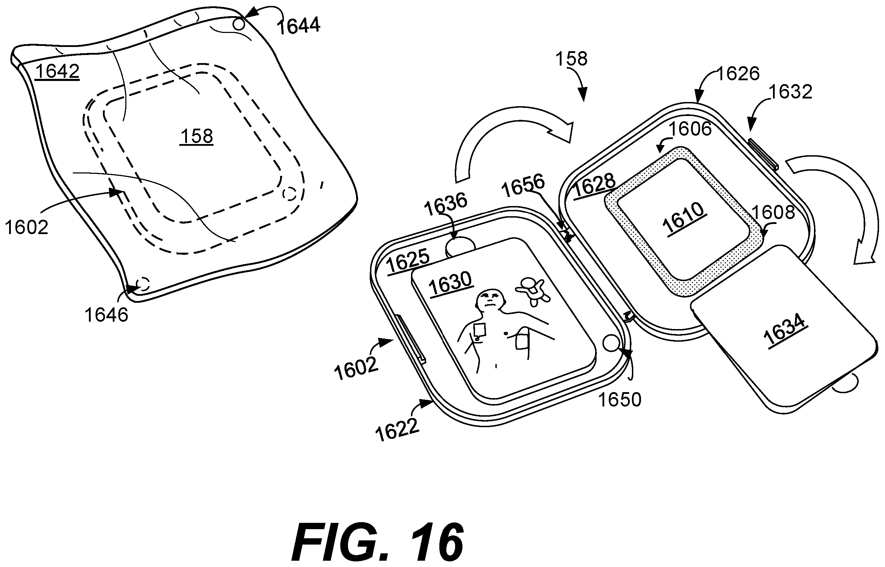

As shown in FIG. 15, the second rigid platform 1502 is in an open configuration. In a closed configuration, it is folded along the midline 1514 of the lateral extension as shown in the exemplary accessory device package 158 in FIG. 16. The second rigid platform 1502 as shown displays and provides access to the accessory devices stored in the interior. In a closed formation, the second rigid platform extends longitudinally between a first opposite end 1518 and a second opposite end 1520 and laterally between a first opposite side 1522 and a second opposite side 1524. The platform may be made of any rigid material including polyvinyl chloride (PVC), acrylic, polyurethane, polycarbonate, polyethylene terephthalate, low-density polyethylene, methacrylate, high-density polyethylene, polystyrene, nylon and the like.

In some aspects, the second rigid platform 1502 may be hinged, for example, there may be a hinge 1556 along the midline 1514. In some aspects, the rigid platform may be maintained under tension while closed, allowing for rapid opening. For example, the hinge 1556 may be spring-loaded. While any type of spring loading may be used, in some aspects the spring loading is accomplished using shape-memory alloy, shape memory polymer, a compression spring, torsion spring, or a technical spring. In some aspects, by maintaining the second rigid platform 1502 under tension when in the closed position, the response time for defibrillation may be decreased.

The open second rigid platform 1502 has a recessed first interior compartment 1525 in a first portion of the second rigid platform and a recessed second interior compartment 1528 in a second portion of the second rigid platform 1502. The outside of the first opposite side 1522 forms a top surface when closed and the outside of the second side 1526 forms a bottom of the device when closed. The two sides may be held closed via a latch 1532 and are connected by the hinge 1556.

An electrode 1510 for an AED may be located in the interior compartments such as recessed first interior compartment 1525 and the second interior compartment 1528. In some aspects, the electrode pad 1506 including the electrode 1510 may be covered with a liner such as foil or other protective covering 1530. Such a protective covering 1530 may include an adhesive or be otherwise removably attached to the electrode pad and/or the bottom of the recessed interior compartments. In some aspects, the covering may include a pictograph showing the correct placement of the electrodes.

In some aspects, the liner may extend the lifespan of the electrode pads it covers. For example, the liner may protect the electrodes from damage prior to use. In some aspects, the electrodes include a dielectric material that is disposed between the skin and the respective electrodes and is configured to enhance the electrical coupling between each electrode and the skin of the subject. The gel, for instance, is a hydrogel. Hydrogels are formed of crosslinking hydrophilic polymers and are capable of retaining water. In various cases, the gel includes one or more electrolytes. In some aspects, the gel may be electrically conductive. The use of a liner such as protective covering 1530, may prevent or decrease the speed at which the electrode pad dries out, extending the life of the electrode pads such as electrode pad 1506. In some aspects, the protective covering 1530 may be peeled away prior to using the electrodes, exposing the electrode pad 1506 as shown and the inside 1534 of the protective covering 1530. For example, a tab, such as tab 1536 may be used to peel the protective covering 1530 such as a foil covering off of the electrode below. Once exposed, the electrode may be used in conjunction with an AED such as AED 136. The electrode pads can be plugged into the AED 136 and attached to the skin of the patient as shown in the pictogram on the protective covering 1530.

After the electrodes are attached to the patient, the AED 136 may be used to monitor a patient. The AED 136 detects an ECG of a patient based on an electrical potential between at least two detection electrodes such as electrode 1510 within an electrode pad 1506. In various examples, the AED 136 evaluates the condition of the patient and displays or otherwise outputs information regarding the patient. In some aspects, the AED 136 may provide spoken or visual instructions as to the next steps to be taken in order to treat an adverse condition of the patient.

According to various implementations, the AED 136 uses the ECG to determine whether a patient is experiencing a shockable heart rhythm, such as VF or pulseless VT. In some examples, AED 136 generates a shock index based on the ECG. The shock index, for example, corresponds to a likelihood that the ECG includes the shockable rhythm. In some cases, a positive shock index indicates that the ECG is more likely than not to include a shockable rhythm, and a negative shock index indicates that the filtered ECG is more likely than not to include a nonshockable rhythm. In some cases, the AED 136 generates the shock index based on one or more parameters of the ECG. For instance, the shock index is based on an amplitude-spectral area (AMSA) value of the ECG.

In various implementations, the AED 136 infers whether a patient is experiencing a shockable rhythm by comparing the shock index to one or more thresholds. For instance, the AED 136 compares the shock index to an upper threshold and a lower threshold. If the shock index exceeds both the upper threshold and the lower threshold, the defibrillator determines that a shockable rhythm is present in the ECG and comes to a "shockable decision." If the shock index is lower than both the upper threshold and the lower threshold, AED 136 determines that a shockable rhythm is not present in the ECG and comes to a "nonshockable decision." If the shock index is between the upper threshold and the lower threshold, the AED 136 comes to an "indeterminate decision." The indeterminate decision means that the AED 136 is unable to conclude whether the shockable rhythm is present with a sufficient level of certainty.

In some cases, the AED 136 automatically begins charging a capacitor upon determining that the shockable rhythm is present, and in some examples automatically begins charging the capacitor when the analysis result is indeterminate. The AED 136 charges a capacitor and/or discharges the capacitor across the defibrillation electrodes, thereby applying a defibrillation shock to the heart of the patient. In some cases, in which the patient is experiencing a shockable arrhythmia, the defibrillation shock may end the shockable arrhythmia and address the source of the cardiac arrest. In some cases, the heart of the patient begins to function effectively, thereby resulting in a return of spontaneous circulation (ROSC).

The components of an AED have various lifespans depending on their composition and design. In their expired state, a medical device and its accessory devices may not be able to perform their intended function which may have serious consequences for patients. For example, if a sufficient amount of water evaporates from a hydrogel coating on an electrode, the electrode may be unable to accurately detect an ECG of a patient or may be unable to safely or effectively administer an electrotherapy (e.g., for the purposes of pacing or defibrillation). In an expired state, the accessory devices may be unable to accurately detect physiological parameters from patients, may be unable to adequately treat patients, or may otherwise malfunction. The lifespan of AED accessory devices, including electrodes in accessory device package158 stored in first rigid platform 102 may be extended by at least partially enclosing them in a sealed pouch as described in further detail with reference to FIG. 16.

Prior to use, the second rigid platform 1602 forming at least in part accessory device package 158, and including accessory devices such as the first electrode pad and the second electrode pad, may be at least partially enclosed in a receptacle such as the sealable pouch 1642 shown in FIG. 16. As shown in FIG. 16, a sealable pouch 1642 may contain a second rigid platform 1602 as shown by the dotted lines, similar to the second rigid platform 1502 of FIG. 15, with the various components in FIG. 16 being similar to the components of FIG. 15 such that the second rigid platform 1602 is configured to contain at least a first electrode pad (beneath protective covering 1630) and a second electrode pad 1606.

The sealable pouch 1642, which may be stored as accessory device package 158, may be opaque, translucent, or transparent and may be made of any flexible or semi-rigid material. In some aspects, the sealable pouch 1642 may be a single-use pouch. In other aspects, the sealable pouch 1642 may be resealable and/or reusable. In some aspects, the sealable pouch 1642 may be vacuum sealed. In some aspects, it may be filled with one or more gasses such as nitrogen, carbon dioxide, oxygen, or argon.

Similarly to FIG. 15, in some aspects, the electrode pads of FIG. 16 may have an adhesive layer 1608 that surrounds the electrode 1610. In some aspects, the electrodes such as a first electrode pad and a second electrode pad 1606 may be connected to wires (not shown) which may extend from one or more connectors configured to plug into an AED 136. The electrodes may be disposable or reusable. For example, they may include an electrically conductive hydrogel on the surface of the electrode or in a gel pack to be applied to the surface of a dry electrode. The hydrogel, for instance, may enhance the electrical coupling between the electrode 1610 and the skin of the patient.

Like FIG. 15, the second rigid platform 1602 shown in FIG. 16 is shown in an open configuration. As in FIG. 15, in a closed configuration, the second rigid platform 1602 is folded along the midline of the lateral extension. When open, the second rigid platform 1602 as shown displays and provides access to the accessory devices stored in the interior. In some aspects, the second rigid platform 1602 may be hinged, for example, there may be a hinge 1656 along the midline. In some aspects, the rigid platform may be maintained under tension while closed, allowing for rapid opening. For example, the hinge 1656 may be spring-loaded. While any type of spring loading may be used, in some aspects the spring loading is accomplished using shape-memory alloy, shape memory polymer, a compression spring, torsion spring, or a technical spring. In some aspects, by maintaining the second rigid platform 1602 under tension when in the closed position, the response time for defibrillation may be decreased.

The open second rigid platform 1602 has a recessed first interior compartment 1625 in a first portion of the second rigid platform and a recessed second interior compartment 1628 in a second portion of the second rigid platform 1602. The outside of the first opposite side 1622 forms a top surface when closed and the outside of the second side 1626 forms a bottom of the device when closed. The two sides may be held closed via a latch 1632 and are connected by the hinge 1656.

An electrode 1610 for an AED may be located in the interior compartments such as recessed first interior compartment 1625 and the second interior compartment 1628. In some aspects, the electrode pad 1606 including the electrode 1610 may be covered with a liner such as foil or other protective covering 1630. Such a protective covering 1630 may include an adhesive or be otherwise removably attached to the electrode pad and/or the bottom of the recessed interior compartments. In some aspects, the covering may include a pictograph showing the correct placement of the electrodes.

In some aspects, the liner may extend the lifespan of the electrode pads it covers. For example, the liner may protect the electrodes from damage prior to use. In some aspects, the electrodes include a dielectric material that is disposed between the skin and the respective electrodes and is configured to enhance the electrical coupling between each electrode and the skin of the subject. The gel, for instance, is a hydrogel. Hydrogels are formed of crosslinking hydrophilic polymers and are capable of retaining water. In various cases, the gel includes one or more electrolytes. In some aspects, the gel may be electrically conductive. The use of a liner such as protective covering 1630, may prevent or decrease the speed at which the electrode pad dries out, extending the life of the electrode pads such as electrode pad 1606. In some aspects, the protective covering 1630 may be peeled away prior to using the electrodes, exposing the electrode pad 1606 as shown and the underside 1634 of the protective covering 1630. For example, a tab, such as tab 1636 may be used to peel the protective covering 1630 such as a foil covering off of the electrode below. Once exposed, the electrode may be used in conjunction with an AED such as AED 136. The electrode pads can be plugged into an AED such as AED 136 and attached to the skin of the patient as shown in the pictogram on the protective covering 1630.

The use of sealable pouches such as sealable pouch 1642 may protect and extend the lifespan of the accessory device(s) stored in the ruggedized defibrillator case within accessory device package 158. For example, the electrode pads such as electrode pads 1606 may contain a dielectric material such as a hydrogel. If a sufficient amount of water evaporates from a hydrogel coating on an electrode pad, the electrode pad may be unable to accurately detect an ECG of a patient or may be unable to safely or effectively administer an electrotherapy (e.g., for the purposes of pacing or defibrillation). The package may assist in decreasing evaporation rates, extending the lifespan of the electrodes.

In some aspects, the sealable pouch 1642 (also referred to herein as a receptacle) may contain one or more sensors on outside and/or the inside surfaces of the pouch. For example, there may be a sensor 1644 on the outside and a sensor 1646 on the inside (shown in dotted line format). The sensors may be the same or different. For example, the sensors may be a humidity sensor, a temperature sensor, a motion sensor or a combination thereof.

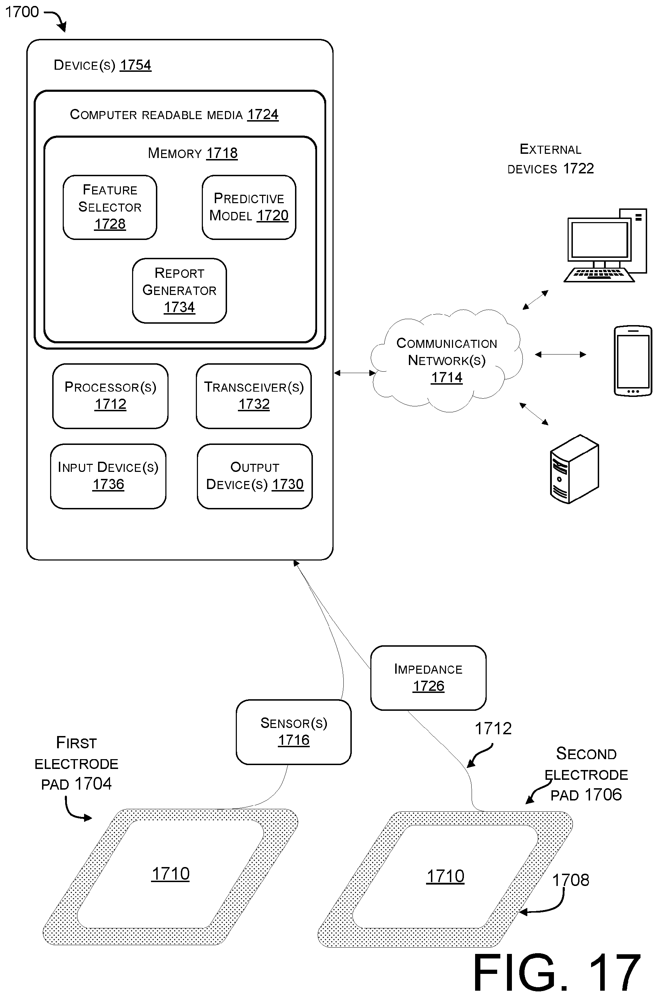

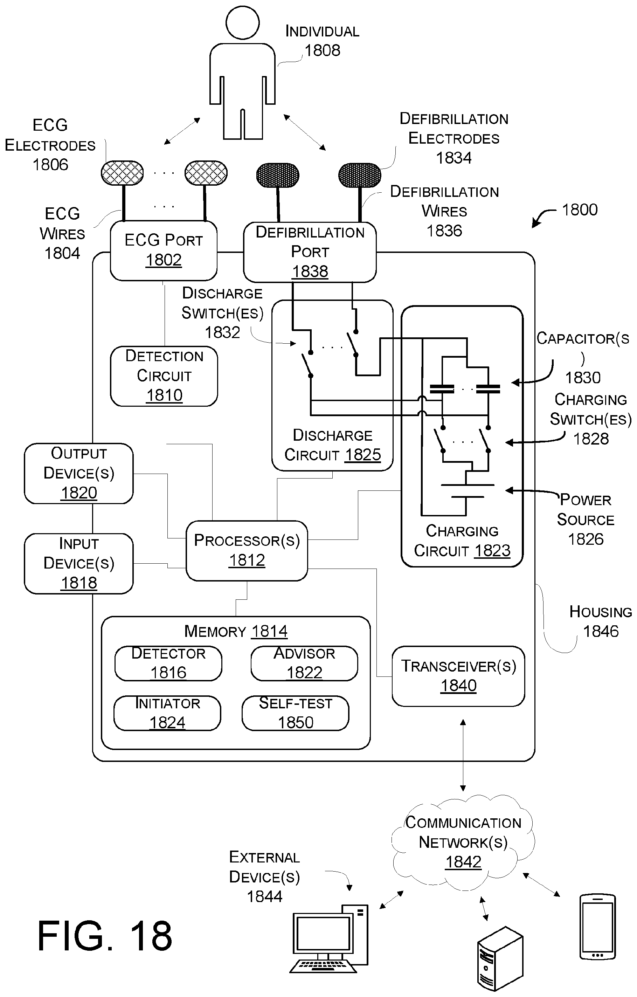

The one or more sensors exemplified by sensor 1550, sensor 1644, sensor 1646, sensor 1650 as well as sensors on the interior or exterior of the ruggedized defibrillator case such as the case shown in FIG. 1, may be designed to measure a variety of conditions that may impact the lifespan of the device. In some aspects, the sensors are environmental sensors that assess chemical, physical, or biological changes in the electrode pads or the conditions under which the electrode pads are maintained. For example, the one or more sensors such as sensor 1550, sensor 1644, and sensor 1646 may measure humidity, temperature, or motion. While the term sensor is used broadly, the sensor may also be an indicator, tag, or hologram. The sensor may detect chemical, enzymatic, mechanical, electrochemical, or microbiological reactions. In some aspects, the sensor is a diode. In other aspects, the sensor is a thermocouple. In other aspects, the sensor may be a position sensor, an RFID chip, an accelerometer, piezoelectric crystals, strain gauges, pressure sensor, force sensor, particle sensor, radiation sensor, electrical sensor, colorimetric sensor, thermochromic ink, or gyroscope. The sensor(s) may convey information regarding the conditions to which the device has been exposed in a variety of ways. For example, it may change color, shape, size, or may communicate with an exterior communication network such as the communication networks shown in FIGs. 17 and 18.

The sensors may provide the same or different types of information and the same or different information as the interior and exterior of the sealable pouch and the device may be exposed to different conditions. For example, the pouch 1642 may be subjected to water but the pouch 1642 remains sealed such that the sensor 1644 conveys water exposure but the sensor 1646 indicates that the second rigid platform 1602 has not been exposed and is still in useable condition. In other examples, the pouch may be breached, but the pouch itself is not exposed to anything damaging. In that scenario, the exterior sensor 1644 would indicate that the pouch is useable, but the interior sensor 1646 would indicate exposure to ambient conditions, that is the interior sensor 1646 would indicate that the contents were no longer under vacuum. There may also be temperature differences between the exterior and the interior of a pouch.

In some aspects, the use of the second rigid platform 1602 may be interrupted or otherwise discontinued. For example, the pouch 1642 may be opened, but the second rigid platform 1602 is not removed or the second rigid platform 1602 is removed from the pouch but not opened, or it is opened, but the protective layer such as protective covering 1630 is not removed. Thus, the various sensors provide different information under different conditions that may impact the lifespan of the rigid platform and the electrodes that reside therein. The measurements obtained by the one or more sensors may then be used in whole or in part to determine the expected lifespan of the accessory device.

The electrodes may be plugged into an external device such as an AED 136 which is shown in more detail with reference to FIG. 17 and FIG. 18. In some aspects, external device(s) may be computing devices including a processor. The processor may be configured to determine an impedance of the electrode gel. Using the impedance of the gel along with information regarding the environmental conditions that the device has been subject to, the external devices may predict the lifespan and/or current state of the accessory device. In some aspects, the predicted lifespan may be within a threshold time period. For example, the original manufacture predicted expiration date may be a threshold time period. In some aspects, the predicted lifespan may be longer than the threshold time period, indicating that the device will last beyond the expected expiration date. In other aspects, the predicted lifespan may be below a threshold time period, indicating the accessory device will expire prior to the expiration date.

FIG. 17 illustrates a system 1700 configured to perform various functions described herein. In various implementations, the operation of the device 1754 (such as an AED) may be controlled by at least one processor 1712. The processor(s) is configured to analyze the signals from the sensor(s) 1716 measuring the environment of the first electrodes 1710 on the first electrode pad 1704 and the second electrode pad 1708. In some aspects, the processor(s) may be configured to analyze the impedance of a gel on the electrodes. The impedance may be determined, for example, using a closed loop test in which a signal is sent through the electrode.

In various implementations, the device 1754 includes at least one transceiver 1732 configured to communicate with at least one external device 1722 over one or more communication networks 1714. Any communication network described herein can be included in the communication network(s) 1714 illustrated in FIG. 17. The external device(s) 1722, for example, includes at least one a subject monitor, a mobile phone, a server, or a computing device. In some implementations, the transceiver(s) 1732 is configured to communicate with the external device(s) 1722 by transmitting and/or receiving signals in a wired fashion and/or wirelessly. For example, the transceiver(s) 1732 includes a NIC, a network adapter, a LAN adapter, or a physical, virtual, or logical address to connect to the various external devices and/or systems. In various examples, the transceiver(s) 1732 includes any sort of wireless transceivers capable of engaging in wireless communication (e.g., RF communication). For example, the communication network(s) 1714 includes one or more wireless networks that include a 3GPP network, such as an LTE RAN (e.g., over one or more LTE bands), an NR RAN (e.g., over one or more NR bands), or a combination thereof. In some cases, the transceiver(s) 1732 includes other wireless modems, such as a modem for engaging in WI-FI^{®}, WIGIG^{®}, WIMAX^{®}, BLUETOOTH^{®}, or infrared communication over the communication network(s) 1714. The signals, in various cases, encode data in the form of data packets, datagrams, or the like.

In various cases, the processor(s) 1712 generates a lifespan based on data encoded in the signals received from the sensors 1716 and impedance measurement 1726. For instance, the signals include impedance measurements and/or environmental conditions and the like, and the processor(s) 1712 outputs a lifespan that can be displayed on one or more of the external devices 1722.

According to some examples, the input device(s) 1736 receives information from one or more sensors 1716. The sensor(s), for example, is configured to detect a physiological parameter of the subject. In some cases, the sensor(s) include one or more one or more accelerometers, one or more humidity sensors, and one or more temperature sensors. The sensor(s), for instance, is configured to detect one or more conditions to which the electrodes have been exposed. According to some implementations, the signals transmitted by the transceiver(s) 1732 indicate the t state parameter(s) of the accessory devices.

The device 1754 further includes at least one output device 1730, in various implementations. Examples of the output device(s) 1730 include, for instance, least one of a display (e.g., a projector, an LED screen, etc.), a speaker, a haptic output device, a printer, a light such as an LED, or any combination thereof. In some implementations, the output device(s) 1730 include a screen configured to display various parameters detected by and/or reported to the device 1754.

The device 1754 further includes memory/storage component 1718 stored in computer readable media 1724. The computer readable media 1724 is illustrated as including memory/storage component 1718. The memory/storage component 1718 represents memory/storage capacity associated with one or more computer-readable media. The memory/storage component 1718 may include volatile media (such as random access memory (RAM)) and/or nonvolatile media (such as read-only memory (ROM), Flash memory, optical disks, magnetic disks, and so forth). The memory/storage component 1718 may include fixed media (e.g., RAM, ROM, a fixed hard drive, and so on) as well as removable media (e.g., Flash memory, a removable hard drive, an optical disc, and so forth). The computer-readable media 1724 may be configured in a variety of other ways as further described below.

The memory/storage component 1718 stores instructions that, when executed by the processor(s) 1712, causes the processor(s) 1712 to perform various operations. In various examples, the memory/storage component 1718 stores methods, threads, processes, applications, objects, modules, any other sort of executable instruction, or a combination thereof. In some cases, the memory/storage component 1718 stores files, databases, or a combination thereof. In various cases, the memory/storage component 1718 stores instructions, programs, threads, objects, data, or any combination thereof, that cause the processor(s) 1712 to perform various functions. In various cases, the memory/storage component 1718 stores one or more parameters that are detected by the device 1754 and/or reported to the device 1754.

In implementations of the present disclosure, the memory/storage component 1718 also stores one or more components including feature selector 1728, predictive model 1720, and report generator 1734. The component(s) include programs, instructions, files, databases, models, or any other type of data that causes the device 1754 to perform any of the functions described herein.

FIG. 18 illustrates an example of an external defibrillator 1800 similar to AED 136 configured to perform various functions described herein using the accessory devices described herein. The external defibrillator 1800 includes an electrocardiogram (ECG) port 1802 connected to multiple ECG wires 1804. In some cases, the ECG wires 1804 are removeable from the ECG port 1802. For instance, the ECG wires 1804 are plugged into the ECG port 1802. The ECG wires 1804 are connected to ECG electrodes 1806, respectively. The ECG electrodes 1806 are similar to electrode pads 1506, 1606, and 1706 as described with reference to FIG. 15, FIG. 16, and FIG. 17. In various implementations, the ECG electrodes 1806 are disposed on different locations on an individual 1808 such as the locations shown in the pictograph on protective covering 1530 of FIG. 15 and 1630 of FIG. 16. A detection circuit 1810 is configured to detect relative voltages between the ECG electrodes 1806. These voltages are indicative of the electrical activity of the heart of the individual 1808.

In various implementations, the ECG electrodes 1806 are in contact with the different locations on the skin of the individual 1808. In some examples, a first one of the ECG electrodes 1806 is placed on the skin between the heart and right arm of the individual 1808, a second one of the ECG electrodes 1806 is placed on the skin between the heart and left arm of the individual 1808, and a third one of the ECG electrodes 1806 is placed on the skin between the heart and a leg (either the left leg or the right leg) of the individual 1808. In these examples, the detection circuit 1810 is configured to measure the relative voltages between the first, second, and third ECG electrodes 1806. Respective pairings of the ECG electrodes 1806 are referred to as "leads" and the voltages between the pairs of ECG electrodes 1806 are known as "lead voltages." In some examples, more than three ECG electrodes 1806 are included, such that 5-lead or 12-lead ECG signals are detected by the detection circuit 1810.

The detection circuit 1810 includes at least one analog circuit, at least one digital circuit, or a combination thereof. The detection circuit 1810 receives the analog electrical signals from the ECG electrodes 1806, via the ECG port 1802 and the ECG wires 1804. In some cases, the detection circuit 1810 includes one or more analog filters configured to filter noise and/or artifact from the electrical signals. The detection circuit 1810 includes an analog-to-digital converter (ADC) in various examples. The detection circuit 1810 generates a digital signal indicative of the analog electrical signals from the ECG electrodes 1806. This digital signal can be referred to as an "ECG signal" or an "ECG."

In some cases, the detection circuit 1810 further detects an electrical impedance between at least one pair of the ECG electrodes 1806. For example, the detection circuit 1810 includes, or otherwise controls, a power source that applies a known voltage (or current) across a pair of the ECG electrodes 1806 and detects a resultant current (or voltage) between the pair of the ECG electrodes 1806. The impedance is generated based on the applied signal (voltage or current) and the resultant signal (current or voltage). In various examples, the detection circuit 1810 includes one or more analog filters configured to filter noise and/or artifact from the resultant signal. The detection circuit 1810 generates a digital signal indicative of the impedance using an ADC. This digital signal can be referred to as an "impedance signal" or an "impedance." In some aspects, the impedance may be the impedance of the electrodes. In other aspects, the impedance may be used to determine if the individual 1808 is exhibiting a shockable rhythm.

The detection circuit 1810 provides the ECG signal and/or the impedance signal to one or more processors 1812 in the external defibrillator 1800. In some implementations, the processor(s) 1812 includes a central processing unit (CPU), a graphics processing unit (GPU), digital signal processing unit (DPU), other processing unit or component known in the art, or any combination thereof.

The processor(s) 1812 is operably connected to memory 1814. In various implementations, the memory 1814 is volatile (such as random access memory (RAM)), nonvolatile (such as read only memory (ROM), flash memory, etc.) or some combination of the two. The memory 1814 stores instructions that, when executed by the processor(s) 1812, causes the processor(s) 1812 to perform various operations. In various examples, the memory 1814 stores methods, threads, processes, applications, objects, modules, any other sort of executable instruction, or a combination thereof. In some cases, the memory 1814 stores files, databases, or a combination thereof. In some examples, the memory 1814 includes, but is not limited to, RAM, ROM, electrically erasable programmable read-only memory (EEPROM), flash memory, or any other memory technology. In some examples, the memory 1814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 1812 and/or the external defibrillator 1800. In some cases, the memory 1814 at least temporarily stores the ECG signal and/or the impedance signal.

In various examples, the memory 1814 includes a detector 1816, which causes the processor(s) 1812 to determine, based on the ECG signal and/or the impedance signal, whether the individual 1808 is exhibiting a particular heart rhythm. For instance, the processor(s) 1812 determines whether the individual 1808 is experiencing a shockable rhythm that is treatable by defibrillation. Examples of shockable rhythms include ventricular fibrillation (VF) and ventricular tachycardia (VT). In some examples, the processor(s) 1812 determines whether any of a variety of different rhythms (e.g., asystole, sinus rhythm, atrial fibrillation (AF), etc.) are present in the ECG signal.

The processor(s) 1812 is operably connected to one or more input devices 1818 and one or more output devices 1820. Collectively, the input device(s) 1818 and the output device(s) 1820 function as an interface between a user and the defibrillator 1800. The input device(s) 1818 is configured to receive an input from a user and includes at least one of a switch, a keypad, a cursor control, a touch-sensitive display, a voice input device (e.g., a microphone), a haptic feedback device (e.g., a gyroscope), or any combination thereof. The output device(s) 1820 includes at least one of a display, a speaker, a haptic output device, a printer, a light indicator such as an LED, or any combination thereof. In various examples, the processor(s) 1812 causes a display among the input device(s) 1818 to visually output a waveform of the ECG signal and/or the impedance signal. In some implementations, the input device(s) 1818 includes one or more touch sensors, the output device(s) 1820 includes a display screen, and the touch sensor(s) are integrated with the display screen. Thus, in some cases, the external defibrillator 1800 includes a touchscreen configured to receive user input signal(s) and visually output physiological parameters, such as the ECG signal and/or the impedance signal.

In some examples, the memory 1814 includes an advisor 1822, which, when executed by the processor(s) 1812, causes the processor(s) 1812 to generate advice and/or control the output device(s) 1820 to output the advice to a user (e.g., a rescuer). In some examples, the processor(s) 1812 provides, or causes the output device(s) 1820 to provide, an instruction to perform CPR on the individual 1808. In some cases, the processor(s) 1812 evaluates, based on the ECG signal, the impedance signal, or other physiological parameters, CPR being performed on the individual 1808 and causes the output device(s) 1820 to provide feedback about the CPR in the instruction. According to some examples, the processor(s) 1812, upon identifying that a shockable rhythm is present in the ECG signal, causes the output device(s) 1820 to output an instruction and/or recommendation to administer a defibrillation shock to the individual 1808.

The memory 1814 also includes an initiator 1825 which, when executed by the processor(s) 1812, causes the processor(s) 1812 to control other elements of the external defibrillator 1800 in order to administer a defibrillation shock to the individual 1808. In some examples, the processor(s) 1812 executing the discharge circuit 1824 selectively causes the administration of the defibrillation shock based on determining that the individual 1808 is exhibiting the shockable rhythm and/or based on an input from a user (received, e.g., by the input device(s) 1818. In some cases, the processor(s) 1812 causes the defibrillation shock to be output at a particular time, which is determined by the processor(s) 1812 based on the ECG signal and/or the impedance signal.

The memory 1814 also includes a trend which receives sensor measurements and impedance measurements to determine the impedance over time. Measurements may be received from a plurality of time points and a model may be applied that predicts the expiration of the accessory device.

The memory 1814 also includes a self-test 1850. The self-test may be configured to cause the device to send a test signal to the electrode to determine the impedance of the electrode gel of the accessory device. The impedance measurement may be used, for example in the trend to determine the expected lifespan of the accessory device. In some aspects, the self-test may be executed by the electrodes such that the electrodes send and receive a signal and use the signal to determine the expected lifespan of the electrode(s). The results of the test signal may be analyzed by a processor such as processor(s) 1812 or by a similar type of processor in the electrode.

The processor(s) 1812 is operably connected to a charging circuit 1823 and a discharge circuit 1824. In various implementations, the charging circuit 1823 includes a power source 1826, one or more charging switches 1828, and one or more capacitors 1830. The power source 1826 includes, for instance, a battery. The processor(s) 1812 initiates a defibrillation shock by causing the power source 1826 to charge at least one capacitor among the capacitor(s) 1830. For example, the processor(s) 1812 activates at least one of the charging switch(es) 1828 in the charging circuit 1823 to complete a first circuit connecting the power source 1826 and the capacitor to be charged. Then, the processor(s) 1812 causes the discharge circuit 1824 to discharge energy stored in the charged capacitor across a pair of defibrillation electrodes 1834, which are in contact with the individual 1808. For example, the processor(s) 1812 deactivates the charging switch(es) 1828 completing the first circuit between the capacitor(s) 1830 and the power source 1826 and activates one or more discharge switches 1832 completing a second circuit connecting the charged capacitor 1830 and at least a portion of the individual 1808 disposed between defibrillation electrodes 1834.

The energy is discharged from the defibrillation electrodes 1834 in the form of a defibrillation shock. For example, the defibrillation electrodes 1834 are connected to the skin of the individual 1808 and located at positions on different sides of the heart of the individual 1808, such that the defibrillation shock is applied across the heart of the individual 1808. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 1832 are controlled by the processor(s) 1812, for example. In various implementations, the defibrillation electrodes 1834 are connected to defibrillation wires 1836. The defibrillation wires 1836 are connected to a defibrillation port 1838, in implementations. According to various examples, the defibrillation wires 1836 are removable from the defibrillation port 1838. For example, the defibrillation wires 1836 are plugged into the defibrillation port 1838. The defibrillation electrodes 1834 may be similar to electrodes 1510, 1610, and 1710.

In various implementations, the processor(s) 1812 is operably connected to one or more transceivers 1840 that transmit and/or receive data over one or more communication networks 1842. For example, the transceiver(s) 1840 includes a network interface card (NIC), a network adapter, a local area network (LAN) adapter, or a physical, virtual, or logical address to connect to the various external devices and/or systems. In various examples, the transceiver(s) 1840 includes any sort of wireless transceivers capable of engaging in wireless communication (e.g., radio frequency (RF) communication). For example, the communication network(s) 1842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 1840 includes other wireless modems, such as a modem for engaging in WI-FI^{®}, WIGIG^{®}, WIMAX^{®}, BLUETOOTH^{®}, or infrared communication over the communication network(s) 1842.

The defibrillator 1800 is configured to transmit and/or receive data (e.g., ECG data, impedance data, data indicative of one or more detected heart rhythms of the individual 1808, data indicative of one or more defibrillation shocks administered to the individual 1808, etc.) with one or more external devices 1844 via the communication network(s) 1842. The external devices 1844 include, for instance, mobile devices (e.g., mobile phones, smart watches, etc.), Internet of Things (IoT) devices, computers (e.g., laptop devices, servers, etc.), or any other type of computing device configured to communicate over the communication network(s) 1842. In some examples, the external device(s) 1844 is located remotely from the defibrillator 1800, such as at a remote clinical environment (e.g., a hospital). According to various implementations, the processor(s) 1812 causes the transceiver(s) 1840 to transmit data to the external device(s) 1844. In some cases, the transceiver(s) 1840 receives data from the external device(s) 1844 and the transceiver(s) 1840 provide the received data to the processor(s) 1812 for further analysis.

In various implementations, the external defibrillator 1800 also includes a housing 1846 that at least partially encloses other elements of the external defibrillator 1800. For example, the housing 1846 encloses the detection circuit 1810, the processor(s) 1812, the memory 1814, the charging circuit 1823, the transceiver(s) 1840, or any combination thereof. In some cases, the input device(s) 1818 and output device(s) 1820 extend from an interior space at least partially surrounded by the housing 1846 through a wall of the housing 1846. In various examples, the housing 1846 acts as a barrier to moisture, electrical interference, and/or dust, thereby protecting various components in the external defibrillator 1800 from damage.

In some implementations, the external defibrillator 1800 is an automated external defibrillator (AED) operated by an untrained user (e.g., a bystander, layperson, etc.) and can be operated in an automatic mode. In automatic mode, the processor(s) 1812 automatically identifies a rhythm in the ECG signal, makes a decision whether to administer a defibrillation shock, charges the capacitor(s) 1830, discharges the capacitor(s) 1830, or any combination thereof. In some cases, the processor(s) 1812 controls the output device(s) 1820 to output (e.g., display) a simplified user interface to the untrained user. For example, the processor(s) 1812 refrains from causing the output device(s) 1820 to display a waveform of the ECG signal and/or the impedance signal to the untrained user, in order to simplify operation of the external defibrillator 1800.

In some examples, the external defibrillator 1800 is a monitor-defibrillator utilized by a trained user (e.g., a clinician, an emergency responder, etc.) and can be operated in a manual mode or the automatic mode. When the external defibrillator 1800 operates in manual mode, the processor(s) 1812 cause the output device(s) 1820 to display a variety of information that may be relevant to the trained user, such as waveforms indicating the ECG data and/or impedance data, notifications about detected heart rhythms, and the like.

### EXAMPLE CLAUSES

The Example Clauses and Example(s) below are included to demonstrate particular implementations of the disclosure. Those of ordinary skill in the art should recognize in light of the present disclosure that many changes can be made to the specific implementations disclosed herein and still obtain a like or similar result without departing from the spirit and scope of the disclosure.

### First Example set

1. A system for storing an automated external defibrillator (AED) and accessory devices, the system including: a first rigid platform extending longitudinally between a first opposite end and a second opposite end and laterally between a first opposite side and a second opposite side, wherein the first rigid platform is configured to be folded along a midline of the longitudinal extension; and a first recessed portion disposed in a first longitudinal half of the first rigid platform and a second recessed portion disposed in a second longitudinal portion of the first rigid platform, the second recessed portion including a space for a defibrillator, wherein an exterior of the first rigid platform includes a light pipe on a first outer surface, a rotating handle, a detachable shoulder strap, or a vent; and wherein an interior of the first rigid platform includes a bracket mount to secure the automated external defibrillator in the second recessed portion or a visually masked storage component in the second recessed portion.
2. The system of clause 1, wherein the first rigid platform has an opening profile of about 145° to about 190°.
3. The system of clause 2, wherein the opening profile is about 169°.
4. The system of any of clauses 1 to 3, further including a mechanism configured to secure accessory devices into the first recessed portion.
5. The system of any of clauses 1 to 4, wherein the exterior of the first rigid platform includes the light pipe on the first outer surface.
6. The system of clause 5, wherein the light pipe is configured to show a status of the AED within the first rigid platform.
7. The system of any of clauses 1 to 6, wherein the exterior of the first rigid platform includes the rotating handle.
8. The system of clause 7, wherein the rotating handle is configured to rotate about 160°to 210°.
9. The system of any of clauses 1 to 8, wherein the rotating handle is attached along the folded midline of the longitudinal extension of the first rigid platform.
10. The system of any of clauses 1 to 9, wherein the rotating handle is attached to a hinge connecting the first longitudinal half and the second longitudinal half of the first rigid platform.
11. The system of any of clauses 1 to 10, wherein the detachable shoulder strap is attached to a removable pin.
12. The system of any of clauses 1 to 11, further including a gasket positioned between the first opposite side and the second opposite side when the first rigid platform is in a closed configuration.
13. The system of any of clauses 1 to 12, wherein the rigid platform further includes a foot, wherein the foot protrudes from a bottom surface of the first rigid platform and parallel to a hinge attached along the folded midline of the longitudinal extension of the first rigid platform.
14. The system of clause 13, wherein the foot includes a non-skid surface.
15. The system of clause 13, wherein the foot is configured to raise a first portion of the first rigid platform, wherein the first portion is adjacent the longitudinal extension of the first rigid platform.
16. The system of any of clauses 1 to 15, wherein the first rigid platform includes the vent and the vent is a protective vent or auto pressure release valve.
17. The system of clause 16, wherein the vent is within the foot.
18. The system of any of clauses 1 to 17, wherein the first rigid platform is waterproof.
19. The system of any of clauses 1 to 18, wherein the first longitudinal half and the second longitudinal half of the rigid platform are configured to be secured together using a latch.
20. The system of clause 19, wherein the latch is spring-loaded.
21. The system of any of clauses 1 to 20, wherein the first rigid platform includes acrylonitrile butadiene styrene, thermoplastic polyurethane, polypropylene, polystyrene, nylon, polycarbonate, polyethylene terephthalate, or methacrylate.
22. The system of any of clauses 1 to 21, wherein the visually masked storage component includes defibrillator accessory devices.
23. The system of clause 22, wherein the accessory devices include: a sealable pouch containing a second rigid platform in a folded configuration, the second rigid platform extending longitudinally between a first opposite end and a second opposite end and laterally between a first opposite side and a second opposite side, the rigid platform being configured to be folded along a midline of the lateral extension; a first recessed portion disposed in a first lateral half of the second rigid platform; a second recessed portion disposed in a second lateral half of the second rigid platform, the first recessed portion including a space for a first accessory and the second recessed portion including a space for a second accessory device; a first foil seal covering the first recessed portion of the second rigid platform and the first accessory device; a second foil seal covering the second recessed portion of the second rigid platform and the second accessory device; and a spring-loaded hinge at the midline of the lateral extension of the second rigid platform.
24. A storage apparatus, including: a rigid platform extending longitudinally between a first opposite end and a second opposite end and laterally between a first opposite side and a second opposite side, wherein the first rigid platform is configured to be folded along a midline of the longitudinal extension; a first recessed portion disposed in a first longitudinal half of the rigid platform and a second recessed portion disposed in a second longitudinal half of the rigid platform, the first recessed portion including a space configured to accommodate a medical device, wherein the exterior of the rigid platform includes a light pipe on a first outer surface, a rotating handle, a detachable shoulder strap, or a vent; and wherein the interior of the first rigid platform includes a bracket mount to secure the medical device in the second recessed portion or a visually masked storage component in the first recessed portion.
25. The storage apparatus of clause 24, wherein the rigid platform has an opening profile of about 145° to about 190°.
26. The storage apparatus of clause 25, wherein the opening profile is 169°.
27. The storage apparatus of any of clauses 24 to 26, further including a mechanism configured to secure accessory devices into the second recessed portion, wherein the mechanism is a snap fit assembly, interference fit assembly, spring loaded, or tension fit assembly.
28. The storage apparatus of any of clauses 24 to 26, wherein the exterior of the first rigid platform includes the light pipe on the first outer surface.
29. The storage apparatus of any of clauses 24 to 28, wherein the light pipe is configured to show a status of the medical device within the first rigid platform.
30. The storage apparatus of any of clauses 24 to 29, wherein the exterior of the first rigid platform includes the rotating handle.
31. The storage apparatus of clause 30, wherein the rotating handle is configured to rotate about 160°to 210°.
32. The storage apparatus of any of clauses 24 to 31, wherein the rotating handle is attached along the folded midline of the longitudinal extension of the first rigid platform.
33. The storage apparatus of any of clauses 24 to 32, wherein the rotating handle is attached to a hinge connecting the first longitudinal half and the second longitudinal half of the first rigid platform.
34. The storage apparatus of any of clauses 24 to 33, wherein the detachable shoulder strap is attached to a removable pin.
35. The storage apparatus of any of clauses 24 to 34, further including a gasket positioned between the first opposite side and the second opposite side when the first rigid platform is in a closed configuration.
36. The storage apparatus of any of clauses 24 to 35, wherein the rigid platform includes a foot, wherein the foot protrudes from a bottom surface of the first rigid platform and parallel to a hinge attached along the folded midline of the longitudinal extension of the first rigid platform.
37. The storage apparatus of clause 36, wherein the foot includes a non-skid surface.
38. The storage apparatus of clause 36, wherein the foot is configured to raise a first portion of the first rigid platform, wherein the first portion is adjacent the longitudinal extension of the first rigid platform.
39. The storage apparatus of any of clauses 24 to 38, wherein the first rigid platform includes the vent.
40. The storage apparatus of clause 39, wherein the vent is within the foot.
41. The storage apparatus of any of clauses 24 to 40, wherein the first rigid platform is waterproof.
42. The storage apparatus of any of clauses 24 to 41, wherein the first longitudinal half and the second longitudinal half of the rigid platform are configured to be secured together using a latch.
43. The storage apparatus of clause 42, wherein the latch is spring-loaded.
44. The storage apparatus of any of clauses 24 to 43, wherein the first rigid platform includes polypropylene, acrylonitrile butadiene styrene, thermoplastic polyurethane, polystyrene, nylon, polycarbonate, polyethylene terephthalate, or methacrylate.
45. The storage apparatus of any of clauses 24 to 44, wherein the visually masked storage component includes defibrillator accessory devices.
46. A method of decreasing response time for defibrillation including: maintaining a spring-loaded first rigid platform in a folded configuration under tension, the spring-loaded first rigid platform extending longitudinally between a first opposite end and a second opposite end and laterally between a first opposite side and a second opposite side, wherein the first rigid platform is configured to be folded along a midline of the longitudinal extension; verifying a status of the defibrillator via a light pipe on a first outer surface of the spring-loaded first rigid platform; releasing the tension of the spring-loaded first rigid platform in a folded configuration, providing access to a defibrillator system located within the folded configuration; and separating a defibrillator from an interior of the spring-loaded first rigid platform.
47. The method of clause 46, wherein when the tension is released, the spring loaded first rigid platform unfolds.
48. The method of clause 47, wherein the spring-loaded first rigid platform unfolds to provide a 169° opening profile.
49. The method of any of clauses 46 to 48, wherein the spring-loaded first rigid platform has a first recessed portion disposed in a first longitudinal half of the spring-loaded first rigid platform and a second recessed portion disposed in a second longitudinal half of the spring-loaded first rigid platform, the second recessed portion including a space for the defibrillator.
50. The method of any of clauses 46 to 48, wherein the spring-loaded first rigid platform has at least one of a quick-detach handle, a detachable shoulder strap, a foot, a non-skid base, and a pressure release valve.
51. The method of any of clauses 46 to 50, wherein the spring-loaded first rigid platform includes a IP68 rated vent.
52. The method of any of clauses 46 to 51, wherein the defibrillator is secured via a bracket mount.
53. The method of clause 52, wherein the defibrillator is configured to be removed from the bracket mount without tools.
54. The method of clause 53, wherein the bracket mount includes a releasable bracket clip.
55. A system including: a package configured to store a medical device system, the package including: a first rigid platform extending longitudinally between a first opposite end and a second opposite end and laterally between a first opposite side and a second opposite side, wherein the first rigid platform is configured to be folded along a midline of the longitudinal extension, the first rigid platform including: a first recessed portion disposed in a first longitudinal half of the first rigid platform; a second recessed portion disposed in a second longitudinal half of the first rigid platform, the second recessed portion including a space for a defibrillator; the exterior of the first rigid platform including a light pipe on a first outer surface and at least one of a rotating handle, a detachable shoulder strap, or a vent; and the interior of the first rigid platform including at least one of a bracket mount to secure the defibrillator and a visually masked storage component; the defibrillator; and a computing device including: a processor configured to determine a status of the defibrillator and output the status to the light pipe on the first outer surface.
56. The system of clause 55 further including: a sealable pouch containing a second rigid platform in a folded configuration; the second rigid platform extending longitudinally between a first opposite end and a second opposite end and laterally between a first opposite side and a second opposite side, wherein the rigid platform is folded along a midline of the lateral extension; a first recessed portion disposed in a first lateral half of the second rigid platform and a second recessed portion disposed in a second lateral half of the second rigid platform, the first recessed portion including a space for a first accessory and the second recessed portion including a space for a second accessory device; a first foil seal covering the first recessed portion and the first accessory device; a second foil seal covering the second recessed portion and the second accessory device; and a spring-loaded hinged portion at the midline of the lateral extension of the rigid platform; wherein the accessory device includes an electrode and an electrode gel including a dielectric material disposed on a surface of the electrode.
57. The system of clause 56, wherein the processor is further configured to determine an impedance of the electrode gel; and predict a lifespan of the accessory device using the impedance of the electrode gel and environmental conditions of the accessory device.
58. The system of clause 56 or 57, wherein the sealable pouch includes a first sensor on an exterior of the sealable pouch, the first sensor configured to detect an environmental condition of the accessory devices.
59. The system of any of clauses 56 to 58, wherein the first rigid platform includes a second sensor on an interior of the rigid platform.
60. The system of clause 59, wherein the second sensor includes a humidity sensor, a temperature sensor, or a motion sensor.
61. The system of any of clauses 56 to 60, wherein the spring-loaded hinged portion of the second rigid platform includes a shape-memory alloy, shape-memory polymer, compression spring, or technical spring.
62. The system of any of clauses 55 to 61, wherein the first rigid platform is waterproof.
63. The system of any of clauses 55 to 62, including the vent, wherein the vent is an IP68 rated vent.
64. A system including: a package for storing a defibrillator system, the package including: a first rigid platform extending longitudinally between a first opposite end and a second opposite end and laterally between a first opposite side and a second opposite side, wherein the first rigid platform is configured to be folded along a midline of the longitudinal extension, the first rigid platform including: a first recessed portion disposed in a first longitudinal portion of the first rigid platform; a second recessed portion disposed in a second longitudinal portion of the first rigid platform, the second recessed portion including a space configured to accommodate a medical device; the exterior of the first rigid platform including a light pipe on a first outer surface and a rotating handle, a detachable shoulder strap, or a rotating handle; and the interior of the first rigid platform including at least one of a bracket mount configured to secure the medical device and a visually masked storage component; the medical device; and a processor configured to: determine a status of the medical device; and output the status to the light pipe on the first outer surface.
65. The system of clause 64 further including: a sealable pouch configured to contain a second rigid platform in a folded configuration; the second rigid platform extending longitudinally between a first opposite end and a second opposite end and laterally between a first opposite side and a second opposite side, wherein the rigid platform is configured to be folded along a midline of the lateral extension; a first recessed portion disposed in a first lateral half of the second rigid platform and a second recessed portion disposed in a second lateral half of the second rigid platform, the first recessed portion including a space configured to accommodate a first accessory and the second recessed portion including a space configured to accommodate a second accessory device; and a spring-loaded hinge at the midline of the lateral extension of the rigid platform.
66. The system of clause 65, wherein the first recessed portion of the first rigid platform includes a visually masked storage compartment, wherein the visually masked storage compartment is configured to hold the sealable pouch.
67. The system of clause 65 or 66, wherein the first accessory device or the second accessory device is a dry electrode.
68. The system of clause 67, wherein the first accessory device or the second accessory device further includes refillable electrode gel packs.
69. The system of clause 68, wherein the refillable electrode gel packs are automatically or manually deployed.
70. The system of clause 69, wherein the electrodes are reusable.
71. The system of any of clauses 65 to 70, further including a first foil seal covering the first recessed portion of the second rigid platform and the first accessory device; and a second foil seal covering the second recessed portion of the second rigid platform and the second accessory device.
72. The system of any of clauses 65 to 71, wherein the first accessory device or the second accessory device further includes an electrode and an electrode gel including a dielectric material disposed on a surface of the electrode.
73. The system of any of clauses 65 to 72, wherein the processor is further configured to determine an impedance of an electrode gel; and predict a lifespan of the accessory device using the impedance of the electrode gel and environmental conditions of the accessory device.
74. The system of any of clauses 65 to 73, wherein the processor is further configured to determine a lifespan of the accessory devices. and output the lifespan to a display.
75. The system of clause 74, wherein the display is the light pipe.
76. The system of any of clauses 65 to 75, wherein the sealable pouch includes a first sensor on an exterior of the sealable pouch, the first sensor configured to detect an environmental condition of the accessory devices.
77. The system of any of clauses 65 to 76, wherein the first rigid platform includes a second sensor on an interior of the first rigid platform.
78. The system of clause 77, wherein the second sensor includes a humidity sensor, a temperature sensor, or a motion sensor.
79. The system of any of clauses 65 to 78, wherein the spring-loaded hinge of the second rigid platform includes a shape-memory alloy, a shape-memory polymer, a compression spring, a double torsion spring or a technical spring.
80. The system of any of clauses 64 to 79, wherein the first rigid platform is waterproof.
81. The system of any of clauses 64 to 80, including a vent, wherein the vent is an IP68 rated vent.
82. The system of any of clauses 64 to 81, wherein the processor is further configured to output the status of the medical device to the light pipe.

### Second Example Set

1. A system for storing an automated external defibrillator (AED) and accessory devices, the system including: a rigid platform extending: longitudinally between a first opposite end and a second opposite end, and laterally between a first opposite side and a second opposite side, the rigid platform including: a first longitudinal half including a first recessed portion; a second longitudinal half including a second recessed portion; a light pipe disposed on an outer surface of the rigid platform, the light pipe being configured to show a status of the AED; and a vent extending between an interior of the rigid platform and an external environment, the rigid platform being configured to be folded along a longitudinal midline; a rotating handle attached to the rigid platform along the longitudinal midline; a detachable shoulder strap attached to the rigid platform along the longitudinal midline; the accessory devices being configured to be disposed in the first recessed portion of the rigid platform; and the AED configured to be secured in the second recessed portion of the rigid platform via a bracket mount.
2. The system of clause 1, wherein the rigid platform has an opening profile of about 145° to about 190°.
3. The system of clause 1 or 2, wherein the system further includes: a visually masked storage component disposed in the first recessed portion of the rigid platform and including a door, the accessory devices being configured to be stored between the door and an interior surface of the first recessed portion of the rigid platform.
4. A system for storing an automated external defibrillator (AED) and accessory devices, the system including: a first rigid platform extending: longitudinally between a first opposite end and a second opposite end, and laterally between a first opposite side and a second opposite side, the first rigid platform including: a first longitudinal half including a first recessed portion; a second longitudinal half including a second recessed portion; the rigid platform being configured to be folded along a longitudinal midline; and a vent extending between an interior of the rigid platform and an external environment; and the accessory devices being configured to be disposed in the first recessed portion of the rigid platform; and the AED configured to be secured in the second recessed portion of the rigid platform via a bracket mount.
5. The system of clause 4 further including a light pipe disposed on an outer surface of the rigid platform, the light pipe being configured to show a status of the AED.
6. The system of clause 4 or 5 further including a rotating handle or detachable shoulder strap attached to the rigid platform along the longitudinal midline.
7. The system of clause 6, wherein the rotating handle is attached to a hinge connecting the first longitudinal half and the second longitudinal half of the first rigid platform.
8. The system of any of clauses 4 to 7, wherein an opening profile of the first rigid platform is about 169°.
9. The system of any of clauses 6 to 8, wherein the detachable shoulder strap is attached via a removable pin.
10. The system of any of clauses 4 to 9, further including a gasket positioned between the first opposite side and the second opposite side when the first rigid platform is in a closed configuration.
11. The system of any of clauses 4 to 10, wherein the rigid platform further includes a foot, wherein the foot protrudes from a bottom surface of the first rigid platform and parallel to a hinge attached along the longitudinal extension of the first rigid platform.
12. The system of clause 11, wherein the vent is within the foot.
13. The system of any of clauses 4 to 13, wherein the vent is a protective vent or auto pressure release valve.
14. The system of any of clauses 4 to 13, wherein the first longitudinal half and the second longitudinal half of the rigid platform are configured to be secured together using a spring-loaded latch.
15. The system of any of clauses 4 to 14, wherein the first rigid platform includes acrylonitrile butadiene styrene, thermoplastic polyurethane, polypropylene, polystyrene, nylon, polycarbonate, polyethylene terephthalate, or methacrylate.
16. The system of any of clauses 4 to 15, further including accessory devices stored in an accessory device package in the first recessed portion, the accessory device package including: a second rigid platform in a folded configuration, the second rigid platform extending longitudinally between a first opposite end and a second opposite end and laterally between a first opposite side and a second opposite side, the second rigid platform being configured to be folded along a midline of the lateral extension; a first recessed portion disposed in a first lateral half of the second rigid platform; a second recessed portion disposed in a second lateral half of the second rigid platform, the first recessed portion of the second rigid platform including a space for a first accessory and the second recessed portion of the second rigid platform including a space for a second accessory device; a first foil seal covering the first recessed portion and the first accessory device in the second rigid platform; a second foil seal covering the second recessed portion and the second accessory device in the second rigid platform; and a spring-loaded hinge at the midline of the lateral extension of the second rigid platform, wherein the accessory device package is enclosed in a sealable pouch.
17. A method of decreasing response time for defibrillation, the method including: enclosing, by a spring-loaded first rigid platform in a folded configuration under tension, a defibrillator, the spring-loaded first rigid platform extending longitudinally between a first opposite end and a second opposite end and laterally between a first opposite side and a second opposite side, wherein the first rigid platform is configured to be folded along a longitudinal midline; outputting, by a light pipe on a first outer surface of the spring-loaded first rigid platform, a status of the defibrillator; releasing the tension of the spring-loaded first rigid platform in a folded configuration, thereby providing access to a defibrillator system located within the folded configuration; and separating, by the spring-loaded first rigid platform, the defibrillator from an interior of the spring-loaded first rigid platform.
18. The method of clause 17, wherein releasing the tension of the spring-loaded first rigid platform includes unfolding the spring-loaded first rigid platform.
19. The method of clause 17 or 18, wherein the spring-loaded first rigid platform has a first recessed portion disposed in a first longitudinal half of the spring-loaded first rigid platform and a second recessed portion disposed in a second longitudinal half of the spring-loaded first rigid platform, the second recessed portion including a space for the defibrillator.
20. The method of any of clauses 17 to 19, wherein the spring-loaded first rigid platform includes at least one of a quick-detach handle, a detachable shoulder strap, a foot, a non-skid base, and a vent.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A system for storing an automated external defibrillator (AED) and accessory devices, the system comprising:
a first rigid platform extending:
longitudinally between a first opposite end and a second opposite end, and
laterally between a first opposite side and a second opposite side;
the first rigid platform comprising:
a first longitudinal half comprising a first recessed portion;
a second longitudinal half comprising a second recessed portion;
the rigid platform being configured to be folded along a longitudinal midline; and
a vent extending between an interior of the rigid platform and an external environment;
the first recessed portion of the first rigid platform being configured to have the accessory devices disposed therein; and
the second recessed portion of the first rigid platform being configured to have the AED secured therein via a bracket mount.

2. The system of claim 1, further comprising a light pipe disposed on an outer surface of the rigid platform, the light pipe being configured to show a status of the AED.

3. The system of claim 1 or 2, further comprising a rotating handle and/or detachable shoulder strap attached to the rigid platform along the longitudinal midline.

4. The system of claim 3, wherein the rotating handle is attached to a hinge connecting the first longitudinal half and the second longitudinal half of the first rigid platform.

5. The system of any of claims 1-4, wherein the first rigid platform has an opening profile of about 145° to about 190°, wherein for instance the opening profile of the first rigid platform is about 169°.

6. The system of claim 3, or any of claims 4 or 5 as far as dependent from claim 3, wherein the detachable shoulder strap is attached via a removable pin.

7. The system of any of claims 1-6, further comprising a gasket positioned between the first opposite side and the second opposite side when the first rigid platform is in a closed configuration.

8. The system of any of claims 1-7, wherein the first rigid platform further comprises a foot, wherein the foot protrudes from a bottom surface of the first rigid platform and parallel to a hinge attached along the longitudinal extension of the first rigid platform, wherein optionally the vent is within the foot.

9. The system of any of claims 1-8, wherein the vent is a protective vent or auto pressure release valve.

10. The system of any of claims 1-9, wherein the first longitudinal half and the second longitudinal half of the rigid platform are configured to be secured together using a spring-loaded latch.

11. The system of any of claims 1-9, wherein the first rigid platform comprises acrylonitrile butadiene styrene, thermoplastic polyurethane, polypropylene, polystyrene, nylon, polycarbonate, polyethylene terephthalate, or methacrylate.

12. The system of any of claims 1-10, wherein the system further comprises:
a visually masked storage component disposed in the first recessed portion of the rigid platform and comprising a door, wherein the system is configured to store the accessory devices between the door and an interior surface of the first recessed portion of the first rigid platform.

13. The system of any of claims 1-11, further comprising accessory devices stored in an accessory device package in the first recessed portion, the accessory device package comprising:
a second rigid platform in a folded configuration, the second rigid platform extending longitudinally between a first opposite end and a second opposite end and laterally between a first opposite side and a second opposite side, the second rigid platform being configured to be folded along a midline of the lateral extension;
a first recessed portion disposed in a first lateral half of the second rigid platform;
a second recessed portion disposed in a second lateral half of the second rigid platform, the first recessed portion of the second rigid platform comprising a space for a first accessory and the second recessed portion of the second rigid platform comprising a space for a second accessory device;
a first foil seal covering the first recessed portion and the first accessory device in the second rigid platform;
a second foil seal covering the second recessed portion and the second accessory device in the second rigid platform; and
a spring-loaded hinge at the midline of the lateral extension of the second rigid platform,
wherein the accessory device package is enclosed in a sealable pouch.

14. A method of decreasing response time for defibrillation, the method comprising:
enclosing, by a spring-loaded first rigid platform in a folded configuration under tension, a defibrillator, the spring-loaded first rigid platform extending longitudinally between a first opposite end and a second opposite end and laterally between a first opposite side and a second opposite side, wherein the first rigid platform is configured to be folded along a longitudinal midline;
outputting, by a light pipe on a first outer surface of the spring-loaded first rigid platform, a status of the defibrillator;
releasing the tension of the spring-loaded first rigid platform in a folded configuration, thereby providing access to a defibrillator system located within the folded configuration; and
separating, by the spring-loaded first rigid platform, the defibrillator from an interior of the spring-loaded first rigid platform.

15. The method of claim 13, wherein releasing the tension of the spring-loaded first rigid platform comprises unfolding the spring-loaded first rigid platform.

16. The method of claim 13 or 14, wherein the spring-loaded first rigid platform has a first recessed portion disposed in a first longitudinal half of the spring-loaded first rigid platform and a second recessed portion disposed in a second longitudinal half of the spring-loaded first rigid platform, the second recessed portion comprising a space for the defibrillator, wherein optionally the spring-loaded first rigid platform comprises at least one of a quick-detach handle, a detachable shoulder strap, a foot, a non-skid base, and a vent.
